# EUROPÄISCHE PATENTANMELDUNG

(11) **EP 1 106 960 A2**
(43) Veröffentlichungstag der Anmeldung: **13.06.2001**
(21) Anmeldenummer: 00125620.5
(22) Anmeldetag: 23.11.2000
(51) Int. Cl.: G01B 3/02

(54) **Messlineal für die Hand- und Armmessung**

(30) Priorität: 08.12.1999 DE 19958939
(71) Anmelder: Jobst GmbH, 46446 Emmerich (DE)
(72) Erfinder: Gattwinkel, Angelika, 33649 Bielefeld (DE); Zimmermann von, Hans A., 46446 Emmerich (DE)
(74) Vertreter: Dinné, Erlend

(57) **Zusammenfassung**

Meßlineal für die Hand- und Armmessung, bestehend aus einer rechteckigen flexiblen Folienkarte, die zumindest an einer der Längskanten eine Linealskalierung aufweist, wobei der Nullpunkt der Linealskalierung um bis zu 5 mm von der Querkante nach innen versetzt angeordnet ist.

## Beschreibung

Die Erfindung betrifft ein Meßlineal, das zum Vermessen der Hand eingesetzt wird, wobei die aus der Messung gewonnenen Daten zur Herstellung eines Kompressionshandschuhs verwendet werden.

Zur Fertigung eines Kompressionshandschuhs mit oder ohne Armteil oder eines Kompressionsstrumpfes müssen vom betreffenden Körperteil gewisse, standardisierte Maße abgenommen werden, die dann die Grundlage zur Anfertigung des Handschuhs beziehungsweise des Strumpfes bilden. Bisher ist zur Längenbestimmung ein Maßband verwendet werden, bevorzugt ein solches mit einer Vorrichtung, die das Maßband selbsttätig in ein Gehäuse zurückzieht.

Es gibt konkrete Meßvorschriften, nach denen vorgegangen wird. Es werden Fixpunkte am betreffenden Körperteil eingezeichnet, deren Abstand anschließend vermessen wird.

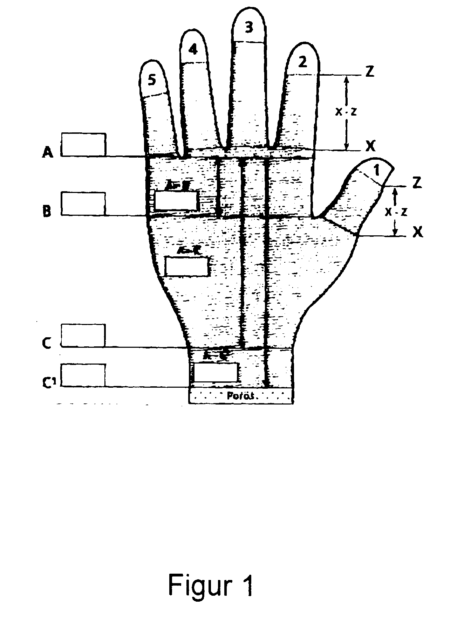

In der Figur 1 ist gezeigt, welche Punkte auf der Hand vorgegeben und welche Maße bestimmt werden.

Die Handinnenfläche des Patienten weist nach oben. Es wird die Innenseite der Hand gemessen, nicht der Handrücken, und die Finger sollten leicht auseinander liegen (wie in der Figur 1). Da die Hand empfindlich auf Druck reagiert, sollte das Maßband nicht zu straff gezogen werden. Die Figur 1 gibt die Meßpunkte an.

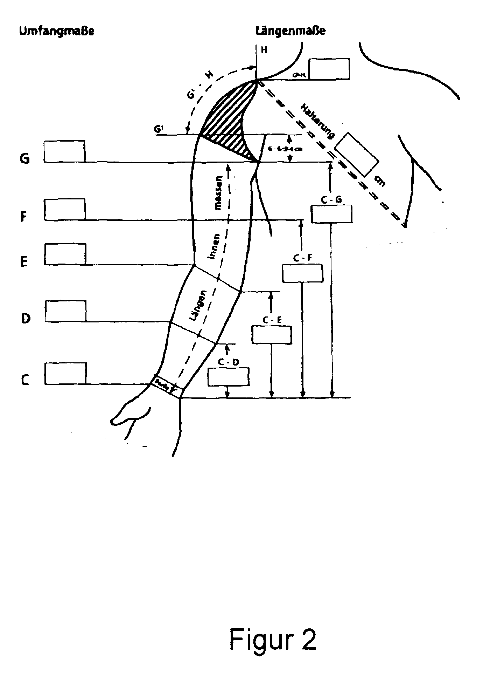

Folgende Abfolge an Meßschritten muß eingehalten werden:
**SCHRITT 1**
   Markierung der Meßpunkte A, B, C und C¹ und Notierung der Längen A-B, A-C und A-C¹.
**SCHRITT 2**
   Messen des Umfangs bei A.
**SCHRITT 3**
   Messen des Umfangs bei B und des Abstandes zwischen den Ebenen A und B.
**SCHRITT 4**
   Messen des Umfangs bei C und des Abstandes zwischen den Ebenen A und C.
**SCHRITT 5**
   Messen des Umfangs bei C¹, also an der Kante des anzufertigenden Handschuhs oder an der proximalen Seite des Speichenknochens oder dort, wo der Handschuh enden soll, sowie des Abstandes zwischen den Ebenen A und C¹.
**SCHRITT 6**
   Für Handschuhe mit Daumen müssen Z und X markiert werden. Es werden dann gemessen der Umfang bei X, die Basis des Daumens, der Umfang bei Z (direkt unterhalb des Knöchels bei Handschuhen mit halbem Daumen oder unterhalb des Daumennagels bei Handschuhen mit ganzem Daumen) sowie das Z-X Maß, welches je nach Länge des Handschuhs variiert.
**SCHRITT 7**
   Wird ein Handschuh mit Fingern benötigt, werden für jeden Finger das Maß X (Umfang an der Basis des Fingers) und das Maß Z (Umfang direkt unterhalb des Knöchels bei Handschuhen mit halbem Finger oder Umfang direkt unterhalb der Fingernägel bei Handschuhen mit ganzem Finger) markiert und gemessen.
**SCHRITT 8**
   Abschließend ist die Fingerlänge zu messen (Z-X). Dieses Maß variiert je nach dem, ob es sich um einen Handschuh mit halbem Finger oder ganzem Finger handelt. Dabei wird Z bei einem Handschuh mit halbem Finger unterhalb des Knöchels und bei einem Handschuh mit ganzem Finger unterhalb des Fingernagels gemessen, und zwar bei geschlossenen Fingern auf der Innenseite der Hand, wobei darauf zu achten ist, daß das Maßband flach auf der Haut aufliegt.
   Das Hauptindikationsgebiet für Armstrümpfe ist die zweite Phase der Lymphödemtherapie mit dem Ziel, das in der ersten Phase der physikalischen Entstauungstherapie erzielte Ergebnis langfristig zu sichern und zu optimieren. Damit der Armstrumpf exakt sitzt und therapeutisch wirksam ist, muß auch dieser genau angemessen werden. In der Figur 2 ist gezeigt, welche Punkte auf dem Arm vorgegeben und welche Maße bestimmt werden.
**SCHRITT 1**
   Markierung der Meßpunkte C, D, E, F, G.
**SCHRITT 2**
   Messen des Umfangs an der Handwurzel bei C und des Umfangs auf Unterarmmitte bei D sowie Bestimmung der Länge C-D von der Handwurzel bis zur Unterarmmitte.
**SCHRITT 3**
   Messen des Umfangs E in der Armbeuge, bei leichter Beugung des Ellenbogengelenks gemessen sowie Bestimmung der Länge C-E von der Handwurzel bis zur Ellenbeuge.
**SCHRITT 4**
   Messen des Umfangs F auf Oberarmmitte sowie Bestimmung der Länge C-F von der Handwurzel bis zur Oberarmmitte.
**SCHRITT 5**
   Messen des Umfangs des Oberarms G ca. 2 Zentimeter unterhalb der Achsel sowie Bestimmung der Länge C-G von der Handwurzel bis 2 Zentimeter unter der Achsel.
   Bei oben abgeschrägten Armstrümpfen werden zusätzlich das G¹-Maß und die Länge C-G¹ ermittelt, bei Armstrümpfen mit Kappe und BH-Befestigung außerdem die Länge G-H:
**SCHRITT 6**
   Zunächst wird um den Oberarm ein weiches, dünnes Kartonstück gelegt. In Abhängigkeit von der Schmerzempfindlichkeit wird der Punkt G bestimmt und damit die Länge des Strumpfes. Der Meßpunkt G¹ liegt etwa 3 bis 4 Zentimeter oberhalb des Meßpunktes G.
**SCHRITT 7**
   Bestimmung der Länge C- G¹, die sich aus der Länge C-G plus etwa 3 bis 4 Zentimeter ergibt.
**SCHRITT 8**
   Bestimmung der Länge G-H, wobei der Meßpunkt H etwa in Schultermitte liegt und den äußeren Rand des BHs bezeichnet. Zusätzlich wird die Trägerbreite vermerkt.

Beim Vermessen einer Hand zur Herstellung eines Kompressionshandschuhes ist auf eine korrekte Längenbestimmung der Finger zu achten. Dies geschieht mittels Anlegen des Nullpunktes eines Maßbandes an den jeweiligen Übergang Fingergrundgelenk zum Handteller. Damit der Kompressionsdruck nicht zu tief in die Schwimmhäute zwischen den Fingern einschneidet beziehungsweise zu früh auf dem Finger endet, ist eine exakte Fingerlängenbestimmung erforderlich.
Ein weiterer Punkt ist der Meßpunkt G am Oberarm, welcher auf Höhe der beginnenden Achselhöhle zu bestimmen ist. Dies muß eine Meßebene sein, die im rechten Winkel aus der Achselhöhle heraus auf die Außenseite des Oberarmes führt. Zur korrekten Bestimmung bedarf es einiger Erfahrung.

Aufgabe der Erfindung ist es, ein Meßlineal zur Verfügung zu stellen, das reproduzierbare Meßwerte garantiert, indem das Meßlineal einfach auf das zu vermessende Körperteil aufgelegt wird.

Gelöst wird diese Aufgabe durch ein Meßlineal, wie es in Anspruch 1 dargelegt ist. Weiterbildungen des erfindungsgemäßen Meßlineal sind dabei Gegenstand der Unteransprüche.

Demgemäß betrifft die Erfindung ein Meßlineal für die Hand- und Armmessung, bestehend aus einer rechteckigen flexiblen Folienkarte, die zumindest an einer der Längskanten eine Linealskalierung aufweist, wobei der Nullpunkt der Linealskalierung um bis zu 5 mm von der Querkante nach innen versetzt angeordnet ist.

Die flexible Folienkarte hat insbesondere die Maße 21 cm x 10 cm. Auf der Karte können des weiteren Informationen u.a. zum Gebrauch der Karte, zum Produkt, zur Indikation angegeben sein.

In einer ersten bevorzugten Ausführungsform ist der Nullpunkt der Linealskalierung um 2 mm nach innen versetzt angeordnet.
Weiterhin ist vorteilhaft, wenn die Einteilung der Linienskalierung in Zentimetern erfolgt.

In einer weiteren bevorzugten Ausführungsform ist auf der Folienkarte auf der Vorder- und auf der Rückseite in gleicher Maßrichtung eine Linealskalierung vorhanden, wobei jeweils der Nullpunkt um bis zu 5 mm von der jeweiligen Querkante nach innen versetzt angeordnet ist.

Zur Bestimmung der Fingerlängen wird die Karte mit dem um bevorzugt 2 mm nach innen versetztem Nullpunkt des Lineals in die Schwimmhaut zwischen den Fingern mit leichtem Druck eingelegt und die Fingerlänge am gewünschten Endpunkt abgelesen. Durch den 2 mm Abstand des Nullpunktes von der Anschlagkante wird ein zu hoher Druck der Naht des fertigen Kompressionshandschuhes auf die Schwimmhaut vermieden. Die Karte dient als Lineal zur Bestimmung der Meßebenen A und B auf der Hand, wobei A die Verbindungslinie zwischen den Fingergrundgelenken Zeigefinger und kleiner Finger und B die rechtwinklige Linie aus Daumenschwimmhaut quer über Handteller darstellt.

Dann lassen sich mit dem Meßlineal hervorragend die Meßpunkte für die Ebenen E und G am Arm bestimmen.

E wird festgelegt durch leichtes Anlegen der Karte in den leicht gebeugten Ellbogenwinkel.
G wird in Abstimmung mit dem Patienten durch Einlegen der Karte im Achselbereich bei angelegtem Arm und anschließendem Umfassen des Oberarms unter Verwendung der Karte bestimmt, die aufgrund ihrer Flexibilität hervorragend den Arm umgibt, was ein einfaches und genaues Markieren der gewünschten Meß-Ebene ermöglicht. Die Mitwirkung des Patienten ist wegen der häufig sehr schmerzempfindlichen Region (Narbengewebe, Strahlenschädigung) von Vorteil.

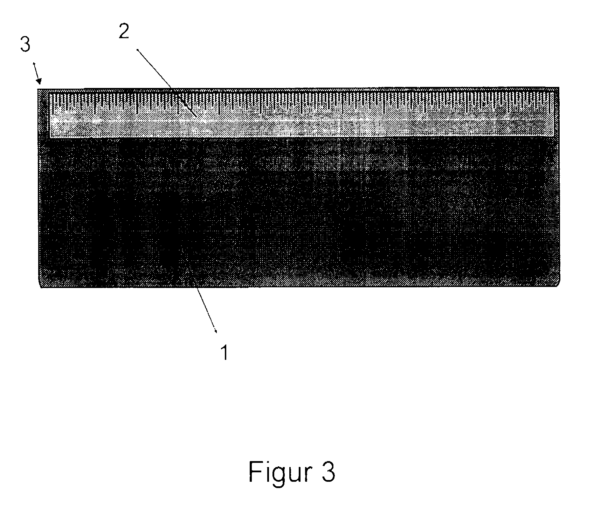

Anhand der Figur 3 soll eine vorteilhafte Ausführungsform des Erfindungsgegenstandes dargestellt werden, ohne damit die Erfindung unnötig beschränken zu wollen.

Die Figur 3 zeigt ein Meßlineal für die Hand- und Armmessung, das aus einer rechteckigen flexiblen Folienkarte 1 besteht. An einer der Längskanten weist die Folienkarte 1 eine Linealskalierung 2 auf. Erfindungsgemäß wird vorgeschlagen, den Nullpunkt 3 der Linealskalierung um 2 mm von der Querkante nach innen versetzt anzuordnen.

Das Meßlineal ist darüber hinaus geeignet, mit weiteren Aufdrucken versehen zu werden, zum Beispiel einer Produktinformation oder den oben geschilderten Meßvorschriften.

## Patentansprüche

1. Meßlineal für die Hand- und Armmessung, bestehend aus einer rechteckigen flexiblen Folienkarte, die zumindest an einer der Längskanten eine Linealskalierung aufweist, wobei der Nullpunkt der Linealskalierung um bis zu 5 mm von der Querkante nach innen versetzt angeordnet ist.

2. Meßlineal nach Anspruch 1, dadurch gekennzeichnet, daß der Nullpunkt der Linealskalierung um 2 mm nach innen versetzt angeordnet ist.

3. Meßlineal nach Anspruch 1, dadurch gekennzeichnet, daß auf der Folienkarte auf der Vorder- und auf der Rückseite in gleicher Maßrichtung eine Linealskalierung vorhanden ist.
